(19) Europäisches Patentamt / European Patent Office / Office européen des brevets

(11) **EP 3 349 809 B1**

(12) **EUROPEAN PATENT SPECIFICATION**

(45) Date of publication and mention
of the grant of the patent:
**06.11.2019 Bulletin 2019/45**

(21) Application number: **16771014.4**

(22) Date of filing: **14.09.2016**

(51) Int Cl.:
***A61L 27/22*** *(2006.01)*

(86) International application number:
**PCT/NL2016/050633**

(87) International publication number:
**WO 2017/048120 (23.03.2017 Gazette 2017/12)**

(54) **BONE VOID FILLING COMPOSITE**

KNOCHENLEEREFÜLLENDE VERBUNDSTOFFMATERIALE

MATÉRIAUX COMPOSÉS POUR LE REMPLISSAGE DE CAVITÉS OSSEUSES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.09.2015 GB 201516179**

(43) Date of publication of application:
**25.07.2018 Bulletin 2018/30**

(73) Proprietor: **Fujifilm Manufacturing Europe B.V.
5047 TK Tilburg (NL)**

(72) Inventors:
• **KLUIJTMANS, Sebastianus Gerardus Johannes
Maria**
**5047 TK Tilburg (NL)**
• **VAN DONGEN, Elisabeth Marianna Wilhelmina
Maria**
**5047 TK Tilburg (NL)**
• **PAWELEC, Kendell Marleen**
**5047 TK Tilburg (NL)**
• **KNYCHALA, Jonathan**
**5047 TK Tilburg (NL)**
• **VERDUIJN, Dennis Adrianus**
**5000 LJ Tilburg (NL)**

(74) Representative: **Revell, Christopher
Intellectual Property Group
FUJIFILM Diosynth Biotechnologies UK Limited
6th Floor, Acresfield
8 - 10 Exchange Street
Manchester M2 7HA (GB)**

(56) References cited:
**WO-A2-2007/040574**

• **H-W KIM ET AL.: "Porous scaffolds of
gelatin-hydroxyapatite nanocomposites
obtained by biomimetic approach:
characterization and antibiotic drug release",
JOURNAL OF BIOMEDICAL MATERIALS
RESEARCH, vol. 74B, no. 2, August 2005
(2005-08), pages 686-698, XP002765145, WILEY,
NEW YORK, NY ISSN: 0021-9304, DOI:
10.1002/jbm.b.30236**
• **S TENG ET AL.: "Formation of
nano-hydroxyapatite in gelatin droplets and the
resulting porous composite microspheres",
JOURNAL OF INORGANIC BIOCHEMISTRY, vol.
101, no. 4, April 2007 (2007-04), pages 686-691,
XP002765146, ELSEVIER INC. ISSN: 0162-0134**
• **S YOUNG ET AL.: "Gelatin as a delivery vehicle
for the controlled release of bioactive
molecules", JOURNAL OF CONTROLLED
RELEASE., vol. 109, no. 1-3, 5 December 2005
(2005-12-05), pages 256-274, XP005196220,
ELSEVIER, AMSTERDAM. ISSN: 0168-3659**

**Description**

The research leading to these results has received funding from the People Programme (Marie Curie Actions) of the European Union's Seventh Framework Programme FP7/2007-2013/ under REA grant agreement n° 607051.

The invention relates to a composites, scaffolds and to their use in medical applications, including fillers for bone voids. Many different materials have been used for bone replacement and substitutes. However, the materials used have not performed as well as natural bone. These bone substitutes have not been ideal because they have very different mechanical properties and often exhibit less than desirable biocompatibility and as such do not exert a high level of control over the process of new bone formation.

One of the recent approaches is described in WO2007040574 where a cross-linked biomimetic nanocomposite is proposed which comprises hydroxyapatite nanocrystals, a natural gelatin and a synthetic polymer. As natural gelatin and another synthetic polymer are used the binding capacities are not easily controlled. Also the use of animal derived components such as gelatin is not preferred.

Another approach is described in US8987204 which describes the administration of specific recombinant gelatins only for inducing the bone generation. This document is silent on the concurrent use of hydroxyapatite which is preferred as a biomimetic and resorbable material for the purpose of scaffolding and bone formation.

Yet another approach using recombinant gelatin is described in JP201302213 where physical mixtures with calcium phosphates are described. This document is however silent with respect to the beneficial biomimetic interaction between calcium phosphate and the recombinant gelatin and hence does not teach how to control this interaction. J. Biomed. Mater. Res. 74B(2), August 2005, pages 686-698 and J. Inorg. Biochem. 101(4), April 2007, pages 686-691 disclose composites of gelatin and hydroxyapatite whose preparation includes a step of co-precipitation occurs. J. Control. Release 109(1-3), 5 December 2005, pages 256-274 lists several composites between gelatin and hydroxyapatite for use in bone regeneration.

According to a first aspect of the present invention there is provided a composite comprising a recombinant gelatin and hydroxyapatite in which the recombinant gelatin comprises glutamic and aspartic acid residues that are distributed homogeneously along a gelatin chain, wherein:

(i) the recombinant gelatin comprises a total of at least 8% glutamic and/or aspartic acids amount per 60 amino acids in row with a standard deviation of at most 1.6;

(ii) the hydroxyapatite is obtained by precipitation in the presence of the recombinant gelatin.

The composites of the present invention improve the efficiency and control of bone formation, e.g. by their use as biomimetic bone void filling composites.

Preferably the standard deviation ($SD_{ED}$) is at most 1.30, more preferably at most 1.10.

The % glutamic and/or aspartic acids amount per 60 amino acids in row may be calculated by dividing the recombinant gelatin into segments, each containing 60 amino acids and, starting at the N-terminus, and disregarding the remainder, dividing the number of glutamic acid (E) and/or (preferably "and") aspartic acid (D) residues by 60 and multiplying the resultant figure by 100%, then calculating the average for all complete rows of 60 in the recombinant gelatin. For example, in the first row of SEQ ID NO:1 shown below there are three E's (glutamic acid residues) and three D's (aspartic acid residues) making a total of six E and D residues and ((6/60) x 100 = 10% in total of glutamic and aspartic acid acids amount per 60 amino acids in a row (5% of E + 5% of D). If one repeats this calculation for all complete rows of 60 in SEQ ID NO:1, one achieves a figure of 9.8% GLU + ASP amount per 60 amino acids in row, as shown in Table 1 below.

Preferably the recombinant gelatin comprises at least 8% in total of glutamic acid and aspartic acids per 60 amino acids in a row, more preferably at least 8% in total of glutamic acid and aspartic acids per 60 amino acids in every complete row of 60 amino acids of the recombinant gelatin starting at the N-terminus of the recombinant gelatin.

The standard deviation ($SD_{ED}$) may be determined as follows: the gelatin chain is divided into segments, each containing 60 amino acids, starting at the N-terminus, and disregarding the remainder. For each of these segments the combined amount of glutamic acid (E) and aspartic acid (D) (collectively $x_i$) is determined and a standard deviation is calculated as follows:

$$SD_{ED} = \sqrt{\frac{\sum_i^n (x_i - \bar{x})^2}{(n-1)}},$$

wherein:

$n$    is the total number of segments containing 60-amino acids in the gelatin;

$x_i$    is the combined amount of glutamic acid (E) and aspartic acid (D) for each segment; and

$$\overline{x} = \frac{\sum_i^n x_i}{n}$$

According to a second aspect of the present invention there is provided a scaffold comprising a composite according to the first aspect of the present invention.

The composites and scaffolds of the present invention offer a high degree of biocompatibility, while exhibiting rapid integration with the surrounding tissues and structures. The scaffold may be any body of matter comprising the composite according to the first aspect of the present invention that can be used for tissue engineering, e.g. in *in vitro* cell culturing or *in vivo* implantation. Typically the scaffold is a shaped, three-dimensional article. Generally the scaffold may be used as the foundation for cells to attach to.

In addition to the composite according to the first aspect of the present invention, the scaffold optionally further contains one or more further ingredients, for example one or more fillers or polymers, for example chitosan, collagen, gelatin, starch, polylactide (PLA), polyglycolide (PGA), poly(lactideglycolide) random copolymer (PLGA), polycaprolactone (PCL), polyethyloxide (PEO) and/or polyethylglycol (PEG), and so forth. In a preferred embodiment, the scaffold according to the second aspect of the present invention is a cross-linked scaffold, e.g. cross-linked by dehydrothermal treatment or by treatment with a crosslinking agent, e.g. hexamethylene diisocyanate or any of the crosslinking agents described below.

In a third aspect of the present invention there is provided a method of preparing a composite according to any one of claims 1 to 4 comprising co-precipitation of hydroxyapatite and the recombinant gelatin, optionally followed by mineralization at a pH between 7.0 and 9.0.

The method for producing the scaffolds of the second aspect of the present invention preferably comprises obtaining hydroxyapatite by precipitation under aqueous conditions in the presence of the recombinant gelatin defined in the first aspect of the present invention, shaping and then drying the precipitate to form a scaffold and optionally crosslinking the scaffold, e.g. by dehydrothermal treatment or by treatment with a chemical cross-linking agent (e.g. as described above).

The precipitation under aqueous conditions may be brought about by, for example, mixing calcium hydroxide, phosphoric acid, and the recombinant gelatin defined in the first aspect of the present invention under aqueous conditions.

In a fourth aspect of the present invention there is provided a method of using a composite according to the first aspect of the present invention (e.g. in the form of a biomimetic nanocomposite), e.g. in bone regeneration therapy. The use preferably comprises implanting the composite according to the first aspect of the present invention, the scaffold according to the second aspect of the present invention or an article comprising the scaffold according to the second aspect of the present invention, into a human or animal body.

In this fourth aspect of the present invention, preferably the scaffold is a cross-linked scaffold.

The details of one or more embodiments of the present invention are set forth in the accompanying drawings and the description below. Other features, objects, and advantages of the invention will be apparent from the description and drawings, and from the claims.

The term "comprising" is to be interpreted as specifying the presence of the stated parts, steps or components, but does not exclude the presence of one or more additional parts, steps or components.

Reference to an element by the indefinite article "a" or "an" does not exclude the possibility that more than one of the element(s) is present, unless the context clearly requires that there be one and only one of the elements. The indefinite article "a" or "an" thus usually means "at least one".

Whereas often the term 'collagen' or the like are also used in the art, the term 'gelatin' will be used throughout the rest of this description. Natural gelatin is a mixture of individual polymers with molecular weights ranging from 5,000 up to more than 400,000 daltons.

"Gelatin" as used herein refers to any gelatin, or to any molecule having at least one structural and/or functional characteristic of gelatin. "Gelatin" includes a single collagen chain, any fragments, derivatives, oligomers, polymers, and subunits thereof, containing at least one collagenous domain (Gly-Xaa-Yaa region, where Xaa and Yaa are independently any amino acid). The term "gelatin" includes engineered sequences not found in nature, e.g. altered collagen sequences, e.g. a collagen sequence that is altered, through deletions, additions, substitutions, or other changes, from a naturally occurring collagen sequence. The terms "recombinant gelatin" and 'gelatin' are used interchangeably.

The terms "RGD sequence" and "RGD motif' are used interchangeably.

The terms "protein" or "polypeptide" or "peptide" are used interchangeably and refer to molecules consisting of a chain of amino acids, without reference to a specific mode of action, size, 3-dimensional structure or origin.

The term "biomimetic" is used to describe the multi-phasic behaviour and material properties and solutions in relation to regenerate natural bone formation by taking inspiration from nature.

The invention will be described for the purposes of illustration only in connection with certain preferred embodiments; however, it is recognized that various changes, modifications, additions and improvements may be made to the illustrated embodiments by those persons skilled in the art, all falling within the spirit and scope of the invention.

DESCRIPTION OF DRAWINGS

Fig. 1: XRD spectrum of a composite according to the present invention (sample 1c described in the Examples below (gelatin/ hydroxyapatite ("HA") composite microspheres comprising highly amorphous HA)) vs sample 1g (gelatin/HA composite microspheres comprising HA that was more crystalline than in Example 1c). In both sample 1c and 1g, the HA had been obtained by precipitation in the presence of the RCP.
Fig. 2: SEM pictures of composite samples sample 1c (=a) and 1g (=b) in the form of microspheres.

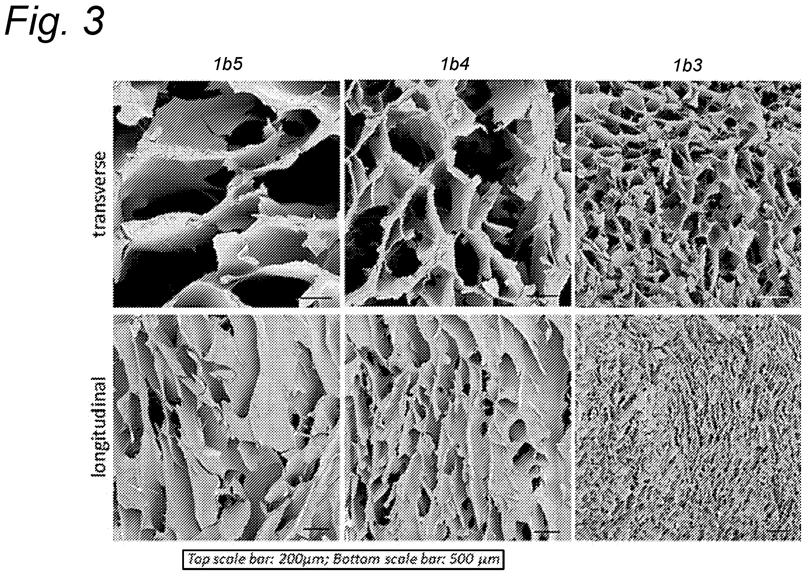

Fig. 3: SEM pictures of cross-sections through composite samples 1b5, 1b4, and 1b3 described in the Examples. These samples are scaffolds of the present invention in the form of anisotropic porous sponges comprising the composite of the first aspect of the present invention. The top pictures are sections in a direction transverse to the pore direction and the bottom pictures are sections in a direction longitudinal to the pore direction.
Fig. 4: FTIR spectra of composite samples 1c (in two concentrations) and 1p.

The recombinant gelatin is preferably a non-fibrilar gelatin and preferably has a lower molecular weight than normal, native gelatin. Furthermore, the recombinant gelatin is further characterised in that it comprises glutamic and/or aspartic acid residues homogeneously distributed along the chain.
The recombinant gelatin comprises a total amount of at least 8% glutamic and/or aspartic acids, e.g. per 60 amino acids in a row, with a standard deviation of at most 1.6. For the purpose of increasing the total HA binding capacity, the absolute occurrence of glutamic and/or aspartic acid residues preferably is at least 9%, more preferably about 10%.
The recombinant gelatin preferably has an average molecular weight of less than 150 kDa, preferably of less than 100 kDa. Preferably the recombinant gelatin has an average molecular weight of at least 5 kDa, preferably at least 10 kDa and more preferably of at least 30 kDa. Preferred average molecular weight ranges for the recombinant gelatin include 50 kDa to 100 kDa, 20 kDa to 75 kDa and 5 kDa to 40 kDa. Lower molecular weights may be preferred when higher mass concentrations of gelatins are required because of the lower viscosity.
The recombinant gelatin may be obtained commercially, e.g. from FUJIFILM under the tradename Cellnest™. The recombinant gelatin may also be prepared, e.g. by known methods, for example as described in patent applications EP 0 926 543 EP1014176. methodology for preparing recombinant gelatins is also described in the publication 'High yield secretion of recombinant gelatins by Pichia pastoris', M. W. T. Werten et al., Yeast 15, 1087-1096 (1999). Suitable recombinant gelatins are also described in WO 2004/85473.
In one embodiment the recombinant gelatin comprises at least two lysine residues, said lysine residues being extreme lysine residues wherein a first extreme lysine residue is the lysine residue that is closest to the N-terminus of the gelatine and the second extreme lysine residue is the lysine residue that is closest to the C-terminus of the gelatine and said extreme lysine residues are separated by at least 25 percent of the total number of amino acids in the gelatin. Such recombinant gelatins may be obtained by, for example, the methods described in US 2009/0246282.
In a preferred embodiment the recombinant gelatin has excellent cell attachment properties and preferably does not display any health-related risks. Advantageously this is achieved by using an RGD-enriched recombinant gelatin, e.g. a recombinant gelatin in which the percentage of RGD motifs related to the total number of amino acids is at least 0.4. If the RGD-enriched gelatin comprises 350 amino acids or more, each stretch of 350 amino acids preferably contains at least one RGD motif. Preferably the percentage of RGD motifs is at least 0.6, more preferably at least 0.8, more preferably at least 1.0, more preferably at least 1.2 and most preferably at least 1.5. A percentage RGD motifs of 0.4 corresponds with at least 1 RGD sequence per 250 amino acids. The number of RGD motifs is an integer, thus to meet the feature of 0.4%, a gelatin consisting of 251 amino acids should comprise at least 2 RGD sequences. Preferably the RGD-enriched recombinant gelatin comprises at least 2 RGD sequences per 250 amino acids, more preferably at least 3 RGD sequences per 250 amino acids, most preferably at least 4 RGD sequences per 250 amino acids. In a further embodiment an RGD-enriched gelatin comprises at least 4 RGD motifs, preferably at least 6, more preferably at least 8, even more preferably at least 12 up to and including 16 RGD motifs.
The recombinant gelatins used in this invention are preferably derived from collagenous sequences. Nucleic acid sequences encoding collagens have been generally described in the art. (See, e. g., Fuller and Boedtker (1981) Biochemistry 20: 996-1006; Sandell et al. (1984) J Biol Chem 259: 7826-34; Kohno et al. (1984) J Biol Chem 259: 13668-13673; French et al. (1985) Gene 39: 311-312; Metsaranta et al. (1991) J Biol Chem 266: 16862-16869; Metsaranta et al. (1991) Biochim Biophys Acta 1089: 241-243; Wood et al. (1987) Gene 61 : 225-230; Glumoff et al. (1994) Biochim Biophys Acta 1217: 41-48 ; Shirai et al. (1998) Matrix Biology 17: 85-88; Tromp et al. (1988) Biochem J 253: 919-912;

Kuivaniemi et al. (1988) Biochem J 252: 633640; and Ala-Kokko et al. (1989) Biochem J 260: 509-516).

Recombinant gelatins enriched in RGD motifs may also be prepared by, for example, the general methods described in US 2006/0241032.

When the composite or scaffold is intended for a pharmaceutical or medical use, the recombinant gelatin preferably has an amino acid sequence which is closely related to or identical to the amino acid sequence of a natural human collagen. More preferably the amino acid sequence of the gelatin comprises repeated amino acid sequences found in native human collagen, especially such a sequence which comprises an RGD motif (in order to create an RGD-enriched recombinant gelatin). The percentage of RGD motifs in such a selected sequence depends on the chosen length of the selected sequence and the selection of a shorter sequence would inevitably result in a higher RGD percentage in the final recombinant gelatin. Repetitive use of a selected amino acid sequence can be used to provide a recombinant gelatin having a higher molecular weight than native gelatin. Furthermore, the recombinant gelatin is preferably non-antigenic and RGD-enriched (compared to native gelatins).

Thus in a preferred embodiment the recombinant gelatin comprises a part of a native human collagen sequence. Preferably the recombinant gelatin is an RGD-enriched gelatin comprising (or consisting of) at least 80% of one or more parts of one or more native human gelatin amino acid sequences. Preferably each of such parts of human gelatin sequences has a length of at least 30 amino acids, more preferably at least 45 amino acids, most preferably at least 60 amino acids, up to e.g. 240, preferably up to 150, most preferably up to 120 amino acids, each part preferably containing one or more RGD sequences. Preferably the RGD-enriched gelatin comprises (or consists of) one or more parts of one or more native human collagen sequences.

An example of a suitable source of recombinant gelatin which may be used in the method of this invention is human COL1A1-1. A part of 250 amino acids comprising an RGD sequence is given in WO 04/85473. RGD sequences in the recombinant gelatin can adhere to specific receptors on cell surfaces called integrins.

RGD-enriched gelatins can be produced by recombinant methods described in, for example, EP-A-0926543, EP-A-1014176 or WO 01/34646, especially in the Examples of the first two mentioned patent publications. The preferred method for producing an RGD-enriched recombinant gelatin comprises starting with a natural nucleic acid sequence encoding a part of the collagen protein that includes an RGD amino acid sequence. By repeating this sequence an RGD-enriched recombinant gelatin may be obtained.

Thus the recombinant gelatins can be produced by expression of nucleic acid sequence encoding such gelatins by a suitable micro-organism. The process can suitably be carried out with a fungal cell or a yeast cell. Suitably the host cell is a high expression host cells like Hansenula, Trichoderma, Aspergillus, Penicillium, Saccharomyces, Kluyveromyces, Neurospora or Pichia. Fungal and yeast cells are preferred to bacteria as they are less susceptible to improper expression of repetitive sequences. Most preferably the host will not have a high level of proteases that cleave the gelatin structure being expressed. In this respect Pichia or Hansenula offers an example of a very suitable expression system. Use of Pichia pastoris as an expression system is disclosed in EP 0 926 543 and EP 1 014 176. The microorganism may be free of active post-translational processing mechanism such as in particular hydroxylation of proline and also hydroxylation of lysine. Alternatively the host system may have an endogenic proline hydroxylation activity by which the gelatin is hydroxylated in a highly effective way.

In a further embodiment, the recombinant gelatin has less glycosylation than native gelatin, e.g. a glycosylation of less than 2 wt%, preferably less than 1 wt%, more preferably less than 0.5 wt%, especially less than 0.2 wt% and more especially less than 0.1 wt%. In a preferred embodiment the recombinant gelatin is free from glycosylation.

The degree or wt% of glycosylation refers to the total carbohydrate weight per unit weight of the gelatin, as determined by, for example, MALDI-TOF-MS (Matrix Assisted Laser Desorption Ionization mass spectrometry) or by the titration method by Dubois. The term 'glycosylation' refers not only to monosaccharides, but also to polysaccharides, e.g. di- tri- and tetra-saccharides.

There are various methods for ensuring that glycosylation is low or absent. Glycosylation is a post-translational modification, whereby carbohydrates are covalently attached to certain amino acids of the gelatin. Thus both the amino acid sequence and the host cell (and enzymes, especially glycosyltransferases) in which the amino acid sequence is produced determine the degree of glycosylation. There are two types of glycosylation: N-glycosylation begins with linking of GlcNAc (N-actylglucosamine) to the amide group of asparagines (N or Asn) and O-glycosylation commonly links GalNAc (N- acetylgalactosamine) to the hydroxyl group of the amino acid serine (S or Ser) or threonine (T or Thr).

Glycosylation can, therefore, be controlled and especially reduced or prevented, by choosing an appropriate expression host, and/or by modifying or choosing sequences which lack consensus sites recognized by the host's glycosyltransferases. Chemical synthesis of gelatin can also be used to prepare gelatin which is free from glycosylation. Also recombinant gelatin which comprises glycosylation may be treated after production to remove all or most of the carbohydrates or non-glycosylated gelatin may be separated from glycosylated gelatin using known methods.

Hydroxyapatite crystals can be formed by combining a calcium and phosphate sources and allowing precipitation. In contrast to homogeneous nucleation for which nucleation takes places randomly in solution, heterogeneously nucleated hydroxyapatite is formed through initial association of the calcium ions with carboxylic acid groups from the aspartic Acid and/or glutamic acid groups on the recombinant gelatin. These crystals may further grow and embed themselves into the matrix structure and thereby mimic the nature of human bone where collagen and hydroxyl apatite are intimately linked.

Surprisingly we found that the recombinant gelatins described in the first aspect of the present invention give rise to efficient nucleation and growth of low-crystalline hydroxyapatite crystals which are associated to the carboxylic acids groups in a biomimetic way (or biomineralization process) which is preferred in terms of resorbability and increased bone formation.

The recombinant gelatins defined in the first aspect of the present invention are advantageously used as they induce efficient mineral nucleation of the hydroxyapatite allowing for a larger mineral binding capacity. Preferably the abovementioned standard deviation is at most 1.3, more preferably at most 1.1.

Hydroxyapatite recombinant gelatin composites can be prepared using methods described in literature for the preparation of collagen / hydroxyapatite composites, for example as described by S. Sprio et al in the Journal of Nanomaterials, Volume 2012, Article ID418281.

One may precipitate hydroxyapatite in the presence of the recombinant gelatin defined in the first aspect of the present invention by, for example, dissolving the recombinant gelatin in an aqueous solution at a concentration typically between 1% and 30%, acidifying the solution using phosphoric acid and mixing this solution with calcium hydroxide, e.g. by adding the acidified recombinant gelatin solution to a solution of calcium hydroxide. It is also possible to first mix the recombinant gelatin with a calcium source (e.g. calcium hydroxide solution) and subsequently add the phosphoric acid.

After precipitating the hydroxyapatite in the presence of the recombinant gelatin (typically allowing a crystallization process to occur in the presence of the recombinant gelatin) a composite slurry is usually obtained. The slurry can be further processed, if desired, by shaping and drying. In this way a scaffold may be formed. Examples of such shaping and drying processes include emulsification, spray drying, moulding, ice templating or freeze drying. Depending on the processing, one may form a scaffold, e.g. a porous or non-porous scaffold. Scaffolds in the form of microspheres are particularly preferred as they may be used to form injectable bone fillers. The microspheres may vary in size and are preferably between 1 and 2000 $\mu$m in diameter, e.g. scaffolds in the form of microspheres having an average diameter of between 1 and 2000 $\mu$m are preferred. Preferably the microspheres are of a size that allows injection into the subject in the need of bone regeneration, e.g. preferred scaffolds are in the form of microspheres having an average diameter of 10 to 200 $\mu$m, e.g. 10, 30, 100 or 200 $\mu$m in diameter.

The use of ice templating techniques, such as described in WO2013068722 allows the formation of scaffolds having anisotropic pore orientations, is also preferred. Anisotropic pore sizes are preferably between 1 to 1000 $\mu$m in diameter. Preferably pore sizes are big enough to allow cell penetration, i.e. at least 10, 30, 100 $\mu$m in diameter. More preferably the scaffold comprises pores of at least 150 $\mu$m (average) in diameter. Preferably the (average) pore size of the scaffold is less than 500$\mu$m in diameter, more preferably less than 450$\mu$m.

The scaffold optionally has a monodisperse or polydisperse pore size distribution. For pore size analysis, preferably at least three SEM micrographs from each scaffold are taken. One may use ImageJ software (ImageJ is a public domain Java image processing program), outlining and then measuring individual pores; the pore size is preferably the average Feret's diameter of at least 40 pores.

To mimic the composition of natural bone, the composites and the scaffolds of the present invention preferably comprise a ratio of hydroxyapatite to the recombinant gelatin between 100:1 and 1:100, more preferably between 10:1 and 1:10 and even more preferably between 5:1 and 1:5. The most preferable ratio of the hydroxyapatite to the recombinant gelatin is 3:2 to 2:3. By selecting the ratio one may achieve good composite stability without sacrificing the chemical cues provided by the hydroxyapatite.

To increase the biomimetic character of the composites and scaffolds of the present invention the hydroxyapatite may further comprise additives such $CO_3^{2-}$, $Na^+$, $Mg^{2+}$, $Sr^{2+}$, $Si^{4+}$, $Zn^{2+}$, $SiO_4^{4-}$ and/or $HPO_4^{2-}$ ions. In one embodiment the composites and scaffolds of the present invention comprise one or more of such additives in a total amount of 0.01% to 25 wt%. Especially preferred are additive concentrations that mimic the amounts of such additives in natural, human bone.

The preferred size of the composites and scaffolds of the present invention depends on the application where the composite is going to be used. For example, the average size of the porous composites and scaffolds may vary from, for example, as small as 1 mm by 1 mm with a thickness of 1 mm to as big as 10 cm by 10 cm with a thickness of 1 cm.

To obtain a residence time of the composite that allows for complete bone regeneration preferably the composites and scaffolds of the present invention are crosslinked. Preferably bone regeneration and composite resorption is a simultaneous process. Crosslinking is preferably achieved using reactive groups present in the recombinant gelatin. Possible ways to cross-link polypeptides are already extensively described in literature. Mostly crosslinking occurs through the carboxylic acid or amine groups of the gelatin.

The crosslinking agent which may be used in the present invention is not particularly limited. For example one may use a chemical crosslinking agent, e.g. formaldehyde, glutaraldehyde, hexamethylene diisocyanate, carbodiimides and/or cyanamide.

Preferably the crosslinking methods used does not impair the biocompatibility of the composite or scaffold and do not generate a strong immune response. In that respect, the use of dehydrothermal treatment as a crosslinking method is preferred. Also the use of hexamethylene diisocyanate as a crosslinking agent is preferred.

The composites and scaffolds of the present invention optionally further comprise excipients which provide a bone filler formulation which further stimulates the bone formation process. Examples of such excipients include synthetic and natural polymers, drugs, growth factors, crosslinkers, natural bone and inorganic components (e.g. calcium phosphates having other crystal structures, tricalcium phosphate, etc.).

The composites and scaffolds of the present invention are particularly useful in the field of bone regeneration, e.g. to fill human bone defects formed by diseases or by trauma. Depending on the site and method of application the composition of the composite or scaffold may need to be adjusted.

The composite and scaffold are preferably in the form of a composition with other ingredients or in the form of a microsphere, particle or sponge. One may use various sizes and shapes for the composite and scaffold appropriate to the bone defect in which they will be placed.

The composite is optionally in the form of an injectable paste or a putty, especially when it is used to fill an irregular-shaped bone defect.

When the composites and scaffolds of the present invention are used as bone filler one may use them in conjunction with other orthopaedic techniques to stabilize bone defects, for example in conjunction with plates and screws. One may mix the composites and scaffolds with a body fluid prior to application as a bone filler, e.g. a body fluid such as blood, blood plasma or bone marrow aspirate.

The invention will now be illustrated by non-liming Examples in which all parts and percentages are by weight unless otherwise specified.

EXAMPLES

Preparation of Recombinant Gelatins

Recombinant gelatins (SEQ ID NO: 1, 2, 3, 4, 5 and 6) were prepared based on a nucleic acid sequence that encodes for a part of the gelatin amino acid sequence of human COLIAI-I and modifying this nucleic acid sequence using the methods disclosed in EP-A-0926543, EP-A-1014176 and WO01/34646. The gelatins did not contain hydroxyproline and comprised the amino acid sequences identified herein as in SEQ ID NO: 1, 2, 3, 4, 5 or 6. The sequences 1 to 5 have the same overall amino acid composition and differ in the distribution of the glutamic (GLU) and aspartic (ASP) acid residues. Except for the last incomplete row, the total amount of GLU + ASP per row of 60 amino acids is shown on the right side of each row.

SEQ ID NO:1:
number of (GLU+ASP) residues per 60 amino acids in a row:

```
GAPGAPGLQGAPGLQGMPGERGADGLPGPKGERGDAGPKGADGAPGAPGLQGMPGERGAA 6
GLPGPKGERGDAGPKGAAGAPGKDGVRGLAGPIGPPGERGAAGLPGPKGERGDAGPKGAD 7
GAPGKDGVRGLAGPIGPPGPAGAPGAPGLQGMPGERGAAGLPGPKGERGDAGPKGADGAP 5
GKDGVRGLAGPPGAPGLQGAPGLQGMPGERGAAGLPGPKGERGDAGPKGADGAPGAPGLQ 5
GMPGERGAAGLPGPKGERGDAGPKGAAGAPGKDGVRGLAGPIGPPGERGAAGLPGPKGER 6
GDAGPKGADGAPGKDGVRGLAGPIGPPGPAGAPGAPGLQGMPGERGAAGLPGPKGERGDA 6
GPKGADGAPGKDGVRGLAGPPGAPGLQGAPGLQGMPGERGAAGLPGPKGERGDAGPKGAD 6
GAPGAPGLQGMPGERGAAGLPGPKGERGDAGPKGADGAPGKDGVRGLAGPIGPPGERGAA 6
GLPGPKGERGDAGPKGADGAPGKDGVRGLAGPIGPPGPAGAPGAPGLQGMPGERGADGLP 6
GPKGERGDAGPKGADGAPGKDGVRGLAGPPG
```

SEQ ID NO:2

```
GAPGAPGLQGAPGLQGMPGERGAAGLPGPKGERGDAGPKGADGAPGAPGLQGMPGERGAA 5
GLPGPKGERGDAGPKGADGAPGKDGVRGLAGPIGPPGERGAAGLPGPKGERGDAGPKGAD 8
GAPGKDGVRGLAGPIGPPGPAGAPGAPGLQGMPGERGAAGLPGPKGERGDAGPKGADGAP 5
GKDGVRGLAGPPGAPGLQGAPGLQGMPGERGAAGLPGPKGERGDAGPKGADGAPGAPGLQ 5
GMPGERGAAGLPGPKGERGDAGPKGADGAPGKDGVRGLAGPIGPPGERGAAGLPGPKGER 7
GDAGPKGADGAPGKDGVRGLAGPIGPPGPAGAPGAPGLQGMPGERGAAGLPGPKGERGDA 6
```

```
GPKGADGAPGKDGVRGLAGPPGAPGLQGAPGLQGMPGERGAAGLPGPKGERGDAGPKGAD 6
GAPGAPGLQGMPGERGAAGLPGPKGERGDAGPKGADGAPGKDGVRGLAGPIGPPGERGAA 6
GLPGPKGERGDAGPKGADGAPGKDGVRGLAGPIGPPGPAGAPGAPGLQGMPGERGAAGLP 5
GPKGERGDAGPKGADGAPGKDGVRGLAGPPG
```

SEQ ID NO:3

```
GAPGAPGLQGAPGLQGMPGERGAAGLPGPKGERGDAGPKGADGAPGAPGLQGMPGERGAA 5
GLPGPKGERGDAGPKGADGAPGKDGVRGLAGPIGPPGERGAAGLPGPKGERGDAGPKGAD 8
GAPGKDGVRGLAGPIGPPGPAGAPGAPGLQGMPGERGAAGLPGPKGERGDAGPKGADGAP 5
GKDGVRGLAGPPGAPGLQGAPGLQGMPGERGAAGLPGPKGERGDAGPKGAAGAPGAPGLQ 4
GMPGERGAAGLPGPKGERGDAGPKGADGAPGKDGVRGLAGPIGPPGERGAAGLPGPKGER 7
GDAGPKGADGAPGKDGVRGLAGPIGPPGPAGAPGAPGLQGMPGERGADGLPGPKGERGDA 7
GPKGADGAPGKDGVRGLAGPPGAPGLQGAPGLQGMPGERGAAGLPGPKGERGDAGPKGAD 6
GAPGAPGLQGMPGERGAAGLPGPKGERGDAGPKGADGAPGKDGVRGLAGPIGPPGERGAA 6
GLPGPKGERGDAGPKGADGAPGKDGVRGLAGPIGPPGPAGAPGAPGLQGMPGERGAAGLP 5
GPKGERGDAGPKGADGAPGKDGVRGLAGPPG
```

SEQ ID NO:4

```
GAPGAPGLQGAPGLQGMPGERGAAGLPGPKGERGDAGPKGADGAPGAPGLQGMPGERGAA 5
GLPGPKGERGDAGPKGADGAPGKDGVRGLAGPIGPPGERGAAGLPGPKGERGDAGPKGAD 8
GAPGKDGVRGLAGPIGPPGPAGAPGAPGLQGMPGERGADGLPGPKGERGDAGPKGADGAP 6
GKAGVRGLAGPPGAPGLQGAPGLQGMPGERGAAGLPGPKGERGDAGPKGAAGAPGAPGLQ 3
GMPGERGAAGLPGPKGERGDAGPKGADGAPGKDGVRGLAGPIGPPGERGADGLPGPKGER 8
GDAGPKGADGAPGKDGVRGLAGPIGPPGPAGAPGAPGLQGMPGERGADGLPGPKGERGDA 7
GPKGADGAPGKDGVRGLAGPPGAPGLQGAPGLQGMPGERGAAGLPGPKGERGDAGPKGAD 6
GAPGAPGLQGMPGERGAAGLPGPKGERGDAGPKGADGAPGKDGVRGLAGPIGPPGERGAA 6
GLPGPKGERGDAGPKGAAGAPGKDGVRGLAGPIGPPGPAGAPGAPGLQGMPGERGAAGLP 4
GPKGERGDAGPKGADGAPGKDGVRGLAGPPG
```

SEQ ID NO:5

```
GAPGAPGLQGAPGLQGMPGERGAAGLPGPKGERGDAGPKGADGAPGAPGLQGMPGERGAA 5
GLPGPKGERGDAGPKGADGAPGKDGVRGLAGPIGPPGERGAAGLPGPKGERGDAGPKGAD 8
GAPGKDGVRGLAGPIGPPGPAGAPGAPGLQGMPGERGADGLPGPKGERGDAGPKGADGAP 6
GKAGVRGLAGPPGAPGLQGAPGLQGMPGERGAAGLPGPKGERGDAGPKGAAGAPGAPGLQ 3
GMPGERGAAGLPGPKGERGDAGPKGADGAPGKDGVRGLAGPIGPPGERGADGLPGPKGER 8
GDAGPKGADGAPGKDGVRGLAGPIGPPGPAGAPGAPGLQGMPGERGADGLPGPKGERGDA 7
GPKGADGAPGKDGVRGLAGPPGAPGLQGAPGLQGMPGERGAAGLPGPKGERGDAGPKGAD 6
GAPGAPGLQGMPGERGAAGLPGPKGVRGDAGPKGADGAPGKDGVRGLAGPIGPPGERGAA 7
GLPGPKGERGDAGPKGAAGAPGKDGERGLAGPIGPPGPAGAPGAPGLQGMPGERGAAGLP 3
GPKGERGDAGPKGADGAPGKDGVRGLAGPPG
```

SEQ ID NO:6

```
GAPGAPGLQGAPGLQGMPGERGAAGLPGPKGERGDAGPKGAAGAPGAPGLQGMPGERGAA 4
GLPGPKGERGDAGPKGAAGAPGKDGVRGLAGPIGPPGERGAAGLPGPKGERGDAGPKGAA 6
GAPGKDGVRGLAGPIGPPGPAGAPGAPGLQGMPGARGADGLPGPKGERGDAGPKGADGAP 5
GKAGVRGLAGPPGAPGLQGAPGLQGMPGARGAAGLPGPKGARGDAGPKGAAGAPGAPGLQ 1
GMPGERGAAGLPGPKGERGDAGPKGAAGAPGKDGVRGLAGPIGPPGERGAAGLPGPKGER 6
GDAGPKGADGAPGKDGVRGLAGPIGPPGPAGAPGAPGLQGMPGERGAAGLPGPKGERGDA 6
GPKGADGAPGKDGVRGLAGPPGAPGLQGAPGLQGMPGERGAAGLPGPKGARGDAGPKGAD 5
GAPGAPGLQGMPGARGAAGLPGPKGERGDAGPKGADGAPGKDGVRGLAGPIGPPGERGAA 5
GLPGPKGERGDAGPKGAAGAPGKAGVRGLAGPIGPPGPAGAPGAPGLQGMPGERGAAGLP 3
GPKGERGDAGPKGADGAPGKDGVRGLAGPPG
```

The distribution of GLU+ASP in the gelatin is represented by the standard deviation of the amounts per row (see Table 1 below). In the amino acid sequences used in the present Examples, the standard deviation gradually increases from 0.6 for SEQ ID NO:1 to 1.9 for SEQ ID NO:5. In addition to a high standard deviation, SEQ ID NO:6 also contains a smaller amount GLU and ASP residues.

SEQ ID NO: 1, 2, 3, 4, 5 and 6 were used to prepare various composites and scaffolds as described below in the examples.

Table 1: Amount/distribution of (GLU +ASP) in SEQ ID NO: 1, 2, 3, 4, 5 and 6.

| Structure | % Amount of (GLU + ASP) | Average Number of (GLU + ASP) residues per 60 amino acids in row | Standard deviation (GLU + ASP) amount per 60 amino acids in row |
|---|---|---|---|
| Inventive Examples | | | |
| SEQ ID NO:1 | 9.8 | 5.9 | 0.60 |
| SEQ ID NO:2 | 9.8 | 5.9 | 1.05 |
| SEQ ID NO:3 | 9.8 | 5.9 | 1.27 |
| Comparative Examples | | | |
| SEQ ID NO: 4 | 9.8 | 5.9 | 1.69 |
| SEQ ID NO: 5 | 9.8 | 5.9 | 1.90 |
| SEQ ID NO:6 | 7.6 | 4.6 | 1.67 |

Example 1) Preparation of Composites by Precipitation of Hydroxyapatite in the Presence of Recombinant Gelatins.

For example Id a solution of 10 grams of gelatin (SEQ ID NO:2) per 100 grams solution was prepared by dissolving the dry gelatin in deionized water. Subsequently phosphoric acid was added (2649 microliters, 86.2m%). This acidic mixture was then added drop-wise into 54.9 grams of a calcium hydroxide suspension containing 4.9 grams calcium hydroxide. The other examples were prepared in a similar way according to the conditions of Table 2.

In some cases the pH was adjusted after precipitation using 1M hydrochloric acid and in one case it was left unadjusted (pH 9, example 1g). The mineralization reaction was then allowed to proceed for 2 hours at ambient conditions. Depending on the added amounts of phosphoric acid and calcium hydroxide in comparison to the gelatin composites having various ratios of gelatin to HA were formed. Examples of mineralization conditions used for inventive examples SEQ ID NO: 1, SEQ ID NO:2 and SEQ ID NO:3 and for Comparative Examples SEQ ID NO:4, SEQ ID NO:5 and SEQ ID NO:6 are shown in Table 2. Furthermore reference sample 1p shown in Table 2 below is also a Comparative Example. This slurry is obtained by mixing calcium phosphate powder (obtained from Sigma-Aldrich) into a solution of gelatin under the conditions shown in Table 2. Thus sample 1p is a physical mixture of calcium phosphate and gelatin, in which the calcium phosphate is not precipitated in the presence of the gelatin. This physical mixing approach is described in JP2013202213.

Table 2: Preparation of Composites

| | gelatin | gelatin concentration (mass%) | pH of mineralization reaction | Ratio gelatin to hydroxyapatite |
|---|---|---|---|---|
| 1a | SEQ ID NO:2 | 2 | 7.2 | 60/40 |
| 1b | SEQ ID NO:2 | 7.5 | 7.2 | 60/40 |
| 1c | SEQ ID NO:2 | 10 | 7.2 | 60/40 |
| 1d | SEQ ID NO:2 | 10 | 7.2 | 40/60 |

(continued)

|  | gelatin | gelatin concentration (mass%) | pH of mineralization reaction | Ratio gelatin to hydroxyapatite |
|---|---|---|---|---|
| 1e | SEQ ID NO:2 | 10 | 7.2 | 80/20 |
| 1f | SEQ ID NO:2 | 10 | 8.0 | 60/40 |
| 1g | SEQ ID NO:2 | 10 | 9.0 | 60/40 |
| 1h | SEQ ID NO:2 | 15 | 7.2 | 60/40 |
| 1i | SEQ ID NO:2 | 20 | 7.2 | 60/40 |
| 1j | SEQ ID NO:1 | 10 | 7.2 | 60/40 |
| 1k | SEQ ID NO:3 | 10 | 7.2 | 60/40 |
| 1l | SEQ ID NO:4 | 10 | 7.2 | 60/40 |
| 1m | SEQ ID NO:5 | 10 | 7.2 | 60/40 |
| 1n | SEQ ID NO:6 | 10 | 7.2 | 60/40 |
| 1p (Physical mixture of gelatin and HA - not co-precipitation) | SEQ ID NO:2 | 10 | 7.2 | 60/40 |

Example 2: formation of scaffolds

The composites obtained as slurries in Example 1 above were further processed to form various scaffolds. Hereafter the formation of (core-shell) microspheres, isotropic and anisotropic sponges are described in the following Examples.

Example 2.1 Microsphere Scaffolds

Samples of 45g of corn oil was pre-warmed at 50°C and stirred at 500 rpm. Then 30g of each the slurries described in Example 1 were added dropwise, in separate experiments, to the corn oil to emulsify for 20 minutes until a volume-weighted average particle size (D[4,3], Malvern Mastersizer 2000) of about 90 $\mu$m was obtained. Then, the resultant emulsions were cooled down to 5°C while stirring and subsequently added into 1.3 times their weight of ice-chilled acetone under stirring to fix the shape and size of the microspheres by water extraction from the cold gelled particles. The resultant microspheres were then washed repeatedly with equal weights of acetone until the microspheres were white and the supernatant clear and colourless. During each acetone wash the microspheres were left to sediment for 10 minutes and the supernatant was decanted-off. The resultant microspheres were then collected by filtration and left to dry overnight at 60°C in a stove.

Subsequently the microspheres were crosslinked by dehydrothermal treatment (48 hours at 160°C under vacuum). The efficacy of the crosslinking was confirmed by a solubility test in which the microsphere scaffolds were put in pH 7.4 saline phosphate buffer at 37 °C for 24 hours. Crosslinking may also be done by hexamethylene diisocyanate crosslinking in ethanol (24 hours, 1% HMDIC in ethanol).

Example 2.2 Core-shell Microsphere Scaffolds

Slurries containing composites were prepared as described in Example 1 and were spray-dried using a Buchi B-290 spray dryer. The resultant particles had a volume-weighted average size (D[4,3], Malvern Mastersizer 2000) of less than 20 micrometers. Before further processing these particles were crosslinked as described above in Example 2.1 Subsequently the crosslinked particles were dispersed in an aqueous phase comprising 10% recombinant gelatin and were again spray-dried. The resulting core-shell particles had a shell consisting of recombinant gelatin and contained one or more core particles comprising both recombinant gelatin and hydroxyapatite in the ratios described in Example 1. Finally these particles were again crosslinked as described above. In another experiment the spray dried gelatin/hydroxyapatite particles with a size of less than 20 micrometer were dispersed in a gelatin/hydroxyapatite slurry as obtained from Example 1 with a gelatin/hydroxyapatite ratio different from the gelatin/hydroxyapatite ratio of the particles. After spray drying and crosslinking core-shell particles were obtained with a core having a different (varying) gelatin/hydroxyapatite ratio than the shell of the microspheres.

Example 2.3 Random or Isotropic Sponge Scaffolds

The slurries of examples 1a-1n were poured into a Teflon coated aluminium container and placed into a pre-cooled lyophilizer (Zirbus 3x4x5) at -20°C for 6 hours to allow complete freezing. Subsequently the samples were lyophilized at a pressure of 0.05 mbar and a temperature of -10°C until dryness. Visual and microscopic inspection of the dry sponges revealed an isotropic and random sponge structure.

Example 2.4 Anisotropic Sponge Scaffolds

The slurries of examples 1b were poured into Teflon coated aluminium containers and subjected to a freezing profile method as described in WO2013068722 to obtain anisotropic sponges. After complete freezing the samples were lyophilized at a pressure of 0.05 mbar and a temperature of -10°C until dryness. Subsequently the sponges were crosslinked as described above in Example 2.1. Dry sponges thus obtained revealed a completely anisotropic pore structure. In a special case the slurry with a composition of example 1b was subjected to various freezing slopes to affect pore size. In this way pore size could be tuned between 80 and 600 micrometer as shown in Table 3. The pore size was the average Feret's diameter of at least 40 pores determined from 3 SEM pictures using ImageJ software. Table 3 also reveals the effect of the pore size on the liquid permeability of the sponges 1b1 - 1b6.

Table 3: Effect of the freezing slope on pore size and liquid permeability of anisotropic sponge scaffolds having composition 1b

| Sample | Freezing slope (°C/minute) | Pore Size (micron) | Liquid permeability in direction along the pores ($10^{-8}$ $m^2$) |
|---|---|---|---|
| 1b1 | 2 | 80 | Not done |
| 1b2 | 1 | 100 | Not done |
| 1b3 | 0.5 | 150 | 0.25 |
| 1b4 | 0.2 | 300 | 0.64 |
| 1b5 | 0.1 | 450 | 2.9 |
| 1b6 | 0.05 | 600 | Not done |

The liquid permeability was measured after crosslinking of the anisotropic sponge scaffolds based on a standard falling-head design. Scaffolds used were 5 mm in diameter, 10 mm long. Prior to testing, scaffolds were pre-wetted with phosphate buffered saline under vacuum. Each measurement was repeated three times to ensure no air bubbles were influencing the results.

Example 3: Effect of Gelatin-type on hydroxyapatite (HA) binding and structure

To analyse the effect of gelatin type on the hydroxyapatite binding and its structure, the gelatin/ hydroxyapatite microsphere scaffolds obtained in Example 2.1 were analysed by scanning electron microscopy (including EDX), FTIR, XRD and TGA as described below.

Scanning electron microscopy

Microsphere scaffolds were fixed on adhesive stubs and coated with a 10 nm thick platinum layer. Images of the microsphere scaffolds were obtained using a Jeol JSM-6335F Field Emission Scanning Electron Microscope. Imaging was carried out at 5 kV voltage, at magnification ranging from x100 to x50 000.

EDX

The microsphere scaffolds were embedded in Leica mounting medium and cross-sections of 0.5, 1 and 2 $\mu$m thickness were cut with a Reichert-Jung Ultracut-E ultra-microtome. Cross sections were coated with 40 nm thick layer of carbon and imaged for calcium & phosphate mapping using an Oxford INCA X-Max 80 detector under 15 kV voltage.

FT-IR

FT-IR analyses were performed using a PerkinElmer Frontier FT-IR Spectrometer. The microsphere scaffolds were squeezed in a diamond compression cell and the spectra were acquired in the range of 4000 to 650 cm$^{-1}$.

Thermo-gravimetric analysis (TGA)

TGA analyses were performed using DSC Mettler Toledo 823e equipped with a gas controller GC10. The experiments were conducted in air and the sample weight was comprised between 8 and 12 mg. The heating was performed in a 70 $\mu$L alumina crucible at a rate of 10°C/min up to 800°C.

X-ray diffraction

X-ray diffraction patterns (XRDs) were recorded by a Bruker AXS D8 Advance instrument in reflection mode (Cu-K$\alpha$ radiation). The samples were ground through a cryo-milling apparatus to obtain relatively uniform particle size powder.

Results

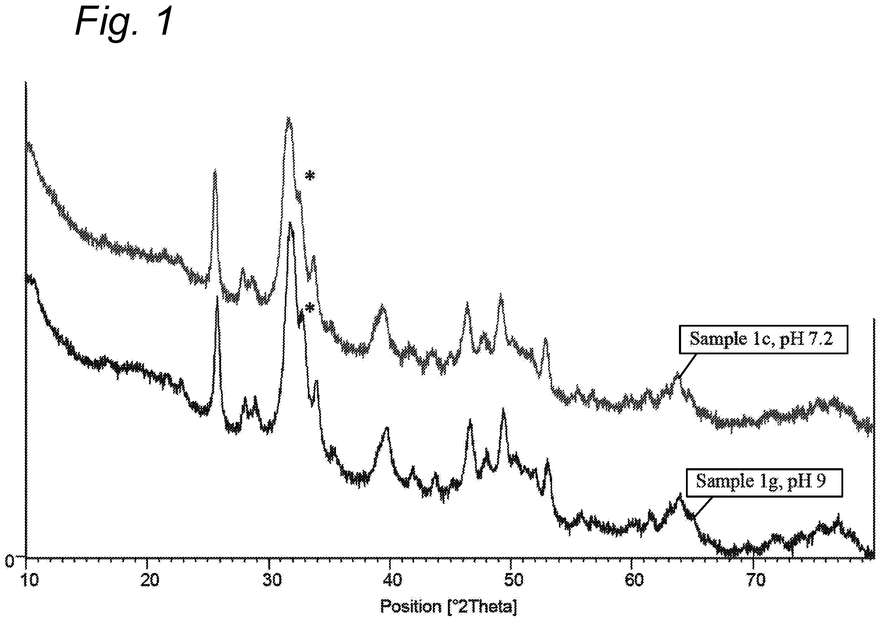

XRD showed that the precipitation step of the present invention resulted in the formation of calcium phosphate in the form of hydroxyapatite, which has been shown to be favourable for bone formation. As an example, the XRD spectrum of composition 1c is shown in Fig. 1. Hydroxyapatite is identified by the characteristic shape of the peaks at 26 and 32 2$\theta$. The small sharpness of the shoulder on the smeared triplet at 32 2$\theta$ (see the asterix in Fig. 1) is indicative of the low crystalline nature of the hydroxyapatite in sample 1c. Low crystallinity enhances the bioresorption of the composite biomaterial and is thus favourable for new bone formation. It is shown in Table 4 that the inventive Examples all have a lower degree of crystallinity than the Comparative Examples as a result of their more homogeneous GLU and/or ASP distribution along the gelatin chain. Also this shoulder is slightly higher when the pH is not adjusted (composite 1g, see Fig. 1). The results further indicate that the preferred direction of pH adjustment when producing the composites of the invention is between 7 and 9 and even more preferred between 7.2 and 8.0.

Table 4: Analysis of the HA crystallization

| Structure | Degree of crystallinity |
|---|---|
| Inventive examples | |
| 1c | Low |
| 1g | Low |
| 1j | Low |
| 1k | Low |
| | |
| Comparative examples | |
| 1l | Moderate |
| 1m | Moderate |

(continued)

| Comparative examples | |
|---|---|
| 1n | Moderate |

The degree of crystallinity was judged from the XRD spectra as described above.

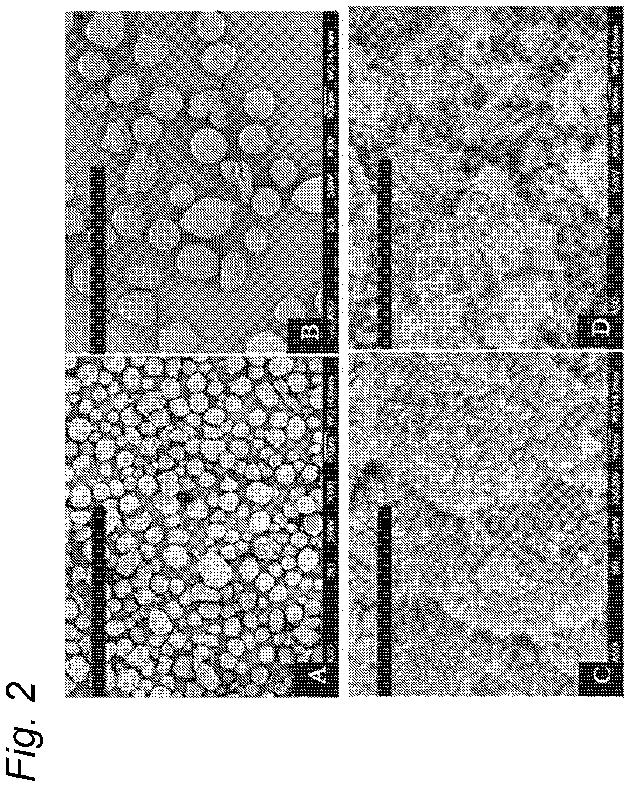

With TGA the actual gelatin/hydroxyapatite weight ratio of the microsphere scaffolds was determined. The measured values were consistent with the amounts of phosphoric acid and calcium hydroxide added in the precipitation reaction. The SEM pictures in Fig. 2 show the rod-shaped morphology of the hydroxyapatite crystals and their nice homogeneous embedding in the gelatin matrix for sample 1c. Comparative example 1l (not shown) clearly shows less crystal embedding and more clustering than the inventive examples. Amongst the inventive examples, the not pH adjusted sample 1g showed the least crystal embedding and largest amount of clustering (see Fig. 2).

By analysing the carbonyl shift in the FTIR analysis it is possible to identify differences in interaction between the organic phase (gelatin biomaterial) and the calcium ions of the inorganic hydroxyapatite phase. When there is no specific interaction between the carbonyl groups of glutamic and aspartic acid and the calcium ions the carbonyl peak is not affected compared to the reference in which just free calcium ions are added. In Table 5 this carbonyl shift is shown for all compositions with a clear difference between the inventive Examples and the Comparative Examples.

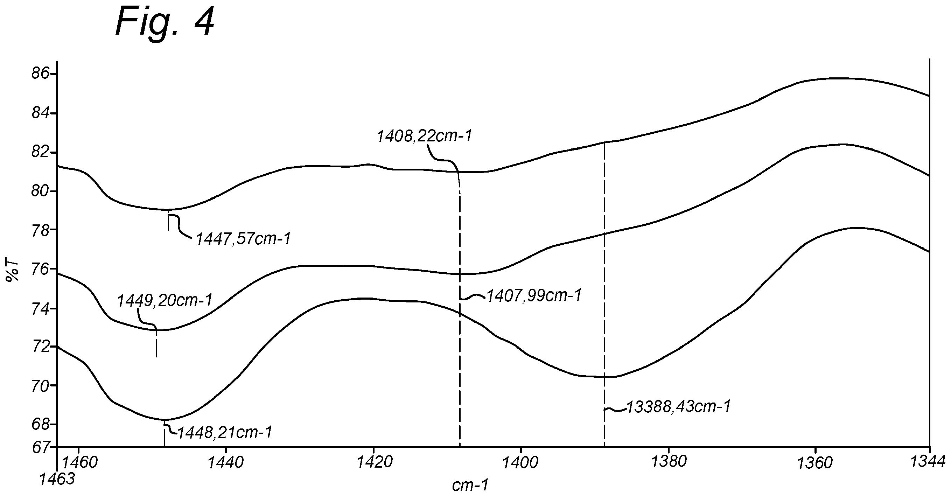

Fig. 4 illustrates the carbonyl shift for samples 1c and 1p in the FTIR spectrum. The unbound Ca reference composition 1p refers to a physical mixture of calcium phosphate and gelatin, in which the calcium phosphate is not precipitated in the presence of gelatin. This physical mixing approach is described in JP2013202213. As evidenced by the strong shift of the carbonyl peak the inventive examples all show a much stronger interaction between the hydroxyapatite or calcium phosphate and carbonyl of the gelatin pointing towards a much more biomimetic character and resemblance to natural bone. Preferably the carbonyl shift of the carboxylic acid group in glutamic and aspartic acid in the microspheres as observed by FTIR is at least 5 cm$^{-1}$ compared to microsphere scaffolds comprising mainly unbound calcium phosphate, more preferably at least 10 cm$^{-1}$, most preferably at least 15 cm$^{-1}$. Also pH adjustment has an effect on the observed peak shift showing the preferred pH adjustment for the composite preparation between 7.0 and 9.0, even more preferred between 7.0 and 8.0.

Table 5: carbonyl peak shift as observed by FTIR as indication for the Ca binding

| Composition | pH | COO Peak position (cm$^{-1}$) |
|---|---|---|
| 1a-1e | 7.2 | 1406 |
| 1f | 8.0 | 1400 |
| 1g | 9.0 | 1397 |
| 1h | 7.2 | 1404 |
| 1i | 7.2 | 1405 |
| 1j | 7.2 | 1403 |
| 1k | 7.2 | 1401 |
| 1l | 7.2 | 1390 |
| 1m | 7.2 | 1391 |
| 1n | 7.2 | 1390 |
| 1p (Physical mixture of gelatin and HA - not co-precipitation) | Not Applicable | 1388 |

In summary, the above analyses indicate that the GLU and/or ASP distribution in the gelatin structure is highly important for the binding of the hydroxyapatite to the gelatin. The GLU and/or ASP distribution influences the crystallinity and crystal-embedding and binding of the resulting crystals to the organic gelatin matrix. The best interaction, resulting in the most biomimetic composite biomaterial was obtained using gelatins in which the amino acids GLU and/or ASP are homogeneously distributed along the amino acid chain. In particular, the biomimetic interaction is best for gelatins having a standard deviation of at most 1.6, preferably at most 1.3. Furthermore it has been shown that the pH during the precipitation reaction is another aspect that is important to affect the interactions between gelatin and hydroxyapatite.

Example 5: Cell culture on Microsphere Scaffolds

C2C12 cells (muscle fibroblast mouse cells CRL-1772 from ATCC) were cultured in routine conditions at 37 °C and 5% $CO_2$ up to 60% confluence in DMEM (Dulbecco's modified eagle's medium from Invitrogen) media supplemented with 10% Foetal Bovine Serum (FBS) (Sigma) and 1% Penicillin-Streptomycin solution x100 (Sigma). Microspheres as obtained in example 2.1 and crosslinked by DHT were seeded in low attachment 24 well plates (Costar, Corning) with 2 mL of cell suspension containing $1x10^5$ cells/mL. The plate was put on an orbital shaker at 30 rpm inside an incubator operating at 37 ° C and 5% $CO_2$ overnight. Following seeding, microspheres were washed with PBS (Phosphate buffered saline from Invitrogen) in order to remove unattached cells and plates were incubated at 37° C and 5% $CO_2$ in static conditions. To analyse cell culturing cells were stained with Live/Dead kit from Invitrogen and were imaged using an Olympus BX60 light microscope. Seeded microspheres were rinsed thoroughly with PBS and incubated with Live/Dead (Invitrogen) mixture for approximately 45 mins in the dark. Thereafter, microspheres were visualised under fluorescent light. The results show that all inventive gelatin/hydroxyapatite microsphere scaffolds are excellent substrates for cells.

Example 6: cell culture on Anisotropic Sponge Scaffolds

Osteoblastic MC3T3-E1 cells (mouse fibroblast CRL-2593 from ATTC) were seeded onto anisotropic scaffolds (of example 2.4 at a density of $5x10^5$ cells per scaffold (5 mm diameter, 2 mm height) using a dynamic shaker method (scaffolds were placed in cell suspension and rotated at 200 rpm for 4 hours, then transferred to cell culture plates in culture media) . Cells were then cultured for 4 weeks in mineralization media. The following scaffolds were used (as prepared in example 2.4): 1b3, 1b4, 1b5. After 4 weeks the amount of cells as determined by DNA quantification (CyQuant Picogreen Assay) was compared to the initial amount of cells attached after 1 day. In table 6 the percentage change in cell numbers is shown. These data show that pore size strongly affects the cell proliferation rate. Based on the hypothesis that cell growth is stimulated by a strong nutrient diffusion one would expect that the largest pore size would give the most cells. However the data imply that there is an optimum at around 300 $\mu$m and a preference pore size of the composite is same or above 150 $\mu$m. It might be speculated that at the larger pore size of 450 $\mu$m there is less pore surface area available for the cells to proliferate which balances the positive effect of the increased nutrient diffusion. The most preferred range of pore size for cell culturing therefore is between 100 and 500 $\mu$m.

Table 6: effect of pore size on the percent change in cell number from day 1 to day 28

| Sample name | Pore Size ($\mu$m) | Percent change in Cell number from 1 to 28 days |
| --- | --- | --- |
| 1b3 | 150 | 1600 |
| 1b4 | 300 | 3250 |
| 1b5 | 450 | 1900 |

**Claims**

1. A composite comprising a recombinant gelatin and hydroxyapatite in which the recombinant gelatin comprises glutamic and aspartic acid residues that are distributed homogeneously along a gelatin chain, wherein:

   (i) the recombinant gelatin comprises a total of at least 8% glutamic and/or aspartic acids amount per 60 amino acids in row with a standard deviation of at most 1.6; and
   (ii) the hydroxyapatite is obtained by precipitation in the presence of the recombinant gelatin.

2. A composite according to claim 1 wherein the hydroxyapatite is obtained by the reaction of phosphoric acid and calcium hydroxide.

3. A composite according to any of the preceding claims wherein the hydroxyapatite further comprises $CO_3^{2-}$, $Na^+$, $Mg^{2+}$, $Sr^{2+}$, $Si^{4+}$, $Zn^{2+}$, $SiO_4^{4-}$ and/or $HPO_4^{2-}$ ions.

4. A composite according to any of the preceding claims wherein the ratio of hydroxyapatite to recombinant gelatin is between 100:1 and 1:100.

5. A composite according to any of the preceding claims which is in the form of microspheres.

**6.** A composite according to claim 5 wherein the microsphere comprises a core and a shell.

**7.** A composite according to claim 6 wherein the shell comprises a different recombinant gelatin/hydroxyapatite ratio to the core.

**8.** A scaffold comprising a composite according to any of the preceding claims.

**9.** A scaffold according to claim 8 wherein the composite is in the form of microspheres and the carbonyl shift of the carboxylic acid group in glutamic and aspartic acid in the microspheres as observed by FTIR is at least 5 cm$^{-1}$ compared to microspheres comprising mainly unbound calcium phosphate.

**10.** A scaffold according to claim 8 in the form of a porous anisotropic sponge.

**11.** The scaffold according to claim 10 wherein the pore size of the pores in the composite is at least 150 $\mu$m.

**12.** A method of preparing a composite according to any one of claims 1 to 7 comprising co-precipitation of hydroxyapatite and the recombinant gelatin, optionally followed by mineralization at a pH between 7.0 and 9.0.

**13.** A composite according to any one of claims 1 to 7 or a scaffold according to any one of claims 8 to 11 for use in bone regeneration therapy.

**14.** A composite according to any one of claims 1 to 7 or a scaffold according to any one of claims 8 to 11 for use in bone regeneration therapy in combination with cells and/or growth factors.

**Patentansprüche**

**1.** Verbundmaterial, umfassend eine rekombinante Gelatine und Hydroxyapatit, bei dem die rekombinante Gelatine Glutaminsäure- und Asparaginsäurereste umfasst, die entlang einer Gelatinekette homogen verteilt sind, wobei:

(i) die rekombinante Gelatine eine Gesamtmenge von wenigstens 8% Glutamin- und/oder Asparaginsäuren pro 60 Aminosäuren in Reihe bei einer Standardabweichung von höchstens 1,6 umfasst; und
(ii) das Hydroxyapatit durch Fällung in Gegenwart der rekombinanten Gelatine erhalten wird.

**2.** Verbundmaterial nach Anspruch 1, wobei das Hydroxyapatit durch die Umsetzung von Phosphorsäure und Calciumhydroxid erhalten wird.

**3.** Verbundmaterial nach einem der vorhergehenden Ansprüche, wobei das Hydroxyapatit ferner $CO_3^{2-}$-, $Na^+$-, $Mg^{2+}$-, $Sr^{2+}$-, $Si^{4+}$-, $Zn^{2+}$-, $SiO_4^{4-}$- und/oder $HPO_4^{2-}$-Ionen umfasst.

**4.** Verbundmaterial nach einem der vorhergehenden Ansprüche, wobei das Verhältnis von Hydroxyapatit zu rekombinanter Gelatine zwischen 100:1 und 1:100 liegt.

**5.** Verbundmaterial nach einem der vorhergehenden Ansprüche, das in Form von Mikrokügelchen vorliegt.

**6.** Verbundmaterial nach Anspruch 5, wobei das Mikrokügelchen einen Kern und eine Hülle umfasst.

**7.** Verbundmaterial nach Anspruch 6, wobei die Hülle ein zum Kern unterschiedliches Rekombinante-Gelatine/Hydroxyapatit-Verhältnis umfasst.

**8.** Gerüst, umfassend ein Verbundmaterial nach einem der vorhergehenden Ansprüche.

**9.** Gerüst nach Anspruch 8, wobei das Verbundmaterial in Form von Mikrokügelchen vorliegt und die Carbonyl-Verschiebung der Carbonsäuregruppe in Glutamin- und Asparaginsäure in den Mikrokügelchen, wie mit FTIR beobachtet, wenigstens 5 cm$^{-1}$ verglichen mit Mikrokügelchen, die hauptsächlich ungebundenes Calciumphosphat umfassen, beträgt.

**10.** Gerüst nach Anspruch 8 in Form eines porösen anisotropen Schwamms.

**11.** Gerüst nach Anspruch 10, wobei die Porengröße der Poren im Verbundmaterial wenigstens 150 μm beträgt.

**12.** Verfahren zur Herstellung eines Verbundmaterials nach einem der Ansprüche 1 bis 7, umfassend gemeinsame Fällung von Hydroxyapatit und der rekombinanten Gelatine, gegebenenfalls gefolgt von Mineralisierung bei einem pH-Wert zwischen 7,0 und 9,0.

**13.** Verbundmaterial nach einem der Ansprüche 1 bis 7 oder Gerüst nach einem der Ansprüche 8 bis 11 zur Verwendung in der Knochenregenerationstherapie.

**14.** Verbundmaterial nach einem der Ansprüche 1 bis 7 oder Gerüst nach einem der Ansprüche 8 bis 11 zur Verwendung in der Knochenregenerationstherapie in Kombination mit Zellen und/oder Wachstumsfaktoren.

## Revendications

**1.** Composite comprenant une gélatine recombinante et de l'hydroxyapatite dans lequel la gélatine recombinante comprend des résidus d'acide glutamique et aspartique qui sont distribués de façon homogène le long d'une chaîne de gélatine, dans lequel :

   (i) la gélatine recombinante comprend un total d'au moins 8 % de quantités d'acides glutamique et/ou aspartique par 60 acides aminés successifs avec un écart type d'au plus 1,6 ; et
   (ii) l'hydroxyapatite est obtenue par précipitation en présence de la gélatine recombinante.

**2.** Composite selon la revendication 1, dans lequel l'hydroxyapatite est obtenue par réaction d'acide phosphorique et d'hydroxyde de calcium.

**3.** Composite selon l'une quelconque des revendications précédentes, dans lequel l'hydroxyapatite comprend en outre des ions $CO_3^{2-}$, $Na^+$, $Mg^{2+}$, $Sr^{2+}$, $Si^{4+}$, $Zn^{2+}$, $SiO_4^{4-}$ et/ou $HPO_4^{2-}$.

**4.** Composite selon l'une quelconque des revendications précédentes, dans lequel le rapport de l'hydroxyapatite à la gélatine recombinante est compris entre 100:1 et 1:100.

**5.** Composite selon l'une quelconque des revendications précédentes qui est sous la forme de microsphères.

**6.** Composite selon la revendication 5, dans lequel la microsphère comprend un noyau et une enveloppe.

**7.** Composite selon la revendication 6, dans lequel l'enveloppe présente un rapport gélatine recombinante/hydroxyapatite différent du noyau.

**8.** Échafaudage comprenant un composite selon l'une quelconque des revendications précédentes.

**9.** Échafaudage selon la revendication 8, dans lequel le composite est sous la forme de microsphères et le déplacement de carbonyle du groupe acide carboxylique dans l'acide glutamique et aspartique dans les microsphères tel qu'observé par FTIR est d'au moins 5 cm$^{-1}$ par rapport à des microsphères comprenant principalement du phosphate de calcium non lié.

**10.** Échafaudage selon la revendication 8, sous la forme d'une éponge anisotrope poreuse.

**11.** Échafaudage selon la revendication 10, dans lequel la taille de pore des pores dans le composite est d'au moins 150 μm.

**12.** Procédé de préparation d'un composite selon l'une quelconque des revendications 1 à 7 comprenant la coprécipitation d'hydroxyapatite et de la gélatine recombinante, facultativement suivie par une minéralisation à un pH compris entre 7,0 et 9,0.

**13.** Utilisation du composite selon l'une quelconque des revendications 1 à 7 ou de l'échafaudage selon l'une quelconque des revendications 8 à 11 pour une thérapie de régénération osseuse.

**14.** Utilisation du composite selon l'une quelconque des revendications 1 à 7 ou de l'échafaudage selon l'une quelconque des revendications 8 à 11 pour une thérapie de régénération osseuse en combinaison avec des cellules et/ou des facteurs de croissance.

Fig. 1

Fig. 2

Fig. 3

Top scale bar: 200μm; Bottom scale bar: 500 μm

EP 3 349 809 B1

Fig. 4

# EP 3 349 809 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007040574 A
- US 8987204 B
- JP 201302213 B
- EP 0926543 A
- EP 1014176 A
- WO 200485473 A
- US 20090246282 A
- US 20060241032 A
- WO 0485473 A
- WO 0134646 A
- WO 2013068722 A
- JP 2013202213 B

### Non-patent literature cited in the description

- *J. Biomed. Mater. Res.,* August 2005, vol. 74B (2), 686-698
- *J. Inorg. Biochem.,* April 2007, vol. 101 (4), 686-691
- *J. Control. Release,* 05 December 2005, vol. 109 (1-3), 256-274
- **M. W. T. WERTEN et al.** High yield secretion of recombinant gelatins by Pichia pastoris. *Yeast,* 1999, vol. 15, 1087-1096
- **FULLER ; BOEDTKER.** *Biochemistry,* 1981, vol. 20, 996-1006
- **SANDELL et al.** *J Biol Chem,* 1984, vol. 259, 7826-34
- **KOHNO et al.** *J Biol Chem,* 1984, vol. 259, 13668-13673
- **FRENCH et al.** *Gene,* 1985, vol. 39, 311-312
- **METSARANTA et al.** *J Biol Chem,* 1991, vol. 266, 16862-16869
- **METSARANTA et al.** *Biochim Biophys Acta,* 1991, vol. 1089, 241-243
- **WOOD et al.** *Gene,* 1987, vol. 61, 225-230
- **GLUMOFF et al.** *Biochim Biophys Acta,* 1994, vol. 1217, 41-48
- **SHIRAI et al.** *Matrix Biology,* 1998, vol. 17, 85-88
- **TROMP et al.** *Biochem J,* 1988, vol. 253, 919-912
- **KUIVANIEMI et al.** *Biochem J,* 1988, vol. 252, 633640
- **ALA-KOKKO et al.** *Biochem J,* 1989, vol. 260, 509-516
- **S. SPRIO et al.** *Journal of Nanomaterials,* vol. 2012